(19) Europäisches Patentamt
European Patent Office
Office européen des brevets

(11) **EP 2 882 500 B1**

(12) **EUROPEAN PATENT SPECIFICATION**

(45) Date of publication and mention of the grant of the patent:
**09.08.2017 Bulletin 2017/32**

(51) Int Cl.:
*A61Q 11/00* (2006.01)   *A61K 8/02* (2006.01)
*A61K 8/19* (2006.01)

(21) Application number: **13729958.2**

(22) Date of filing: **20.06.2013**

(86) International application number:
**PCT/EP2013/062829**

(87) International publication number:
**WO 2014/023465 (13.02.2014 Gazette 2014/07)**

(54) **COMPOSITIONS FOR TREATING TOOTH HYPERSENSITIVITY**

ZUSAMMENSETZUNGEN ZUR BEHANDLUNG VON ZAHNÜBEREMPFINDLICHKEIT

COMPOSITIONS POUR TRAITER L'HYPERSENSIBILITÉ DENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.08.2012 EP 12179880**

(43) Date of publication of application:
**17.06.2015 Bulletin 2015/25**

(73) Proprietors:
• **Unilever PLC**
  **London, Greater London EC4Y 0DY (GB)**
  Designated Contracting States:
  **CY GB IE MT**
• **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventor: **ASHCROFT, Alexander, Thomas**
  **Bebington**
  **Wirral**
  **Merseyside CH63 3JW (GB)**

(74) Representative: **Tansley, Sally Elizabeth et al**
  **Unilever PLC**
  **Unilever Patent Group**
  **Colworth House**
  **Sharnbrook**
  **Bedford**
  **Bedfordshire MK44 1LQ (GB)**

(56) References cited:
**EP-A1- 2 438 901**   **WO-A1-00/78270**
**WO-A1-2012/057739**   **WO-A2-2008/005509**
**US-A- 5 882 631**   **US-A1- 2004 161 388**
**US-A1- 2005 106 110**   **US-A1- 2009 202 451**

Remarks:
The file contains technical information submitted after the application was filed and not included in this specification

**Description**

Field of the Invention

The present invention relates to methods for treating tooth hypersensitivity using certain particulate calcium carbonates.

Background and Prior Art

Tooth hypersensitivity is a common but painful condition affecting up to 20% of the adult population. Hypersensitive teeth may be sensitive to cold, heat, air or sugary foods.

The incidence of tooth hypersensitivity increases with age. Tooth hypersensitivity is believed to be related to the general increase in exposed root surfaces of teeth as a result of periodontal disease, toothbrush abrasion, or cyclic loading fatigue of the thin enamel near the dento-enamel junction. When root surfaces are exposed, dentinal tubules are also exposed. Dentinal tubules are naturally present in the dentinal layer of the tooth and they provide for an osmotic flow between the inner pulp region of the tooth and the outer root surfaces.

The currently accepted theory for tooth hypersensitivity is the hydrodynamic theory, based on the belief that open exposed dentinal tubules allow fluid flow through the tubules. This flow excites the nerve endings in the dental pulp. Clinical replicas of sensitive teeth viewed in a scanning electron microscope (SEM) reveal varying numbers of open or partially occluded dentinal tubules.

There are two categories of therapy for the treatment of tooth hypersensitivity based upon two modes of action. The first category, nerve-depolarising agents, are pharmaceutical agents such as potassium nitrate, which function by interfering with neural transduction of the pain stimulus.

The second category, known as occluding agents, function by physically blocking the exposed ends of the dentinal tubules, thereby reducing dentinal fluid movement and reducing the irritation associated with the shear stress described by the hydrodynamic theory.

An example of an occluding agent is found in US 5,270,031 which describes a tubule occluding desensitizer comprising a polyacrylic acid such as Carbopol® polymeric materials. Another tubule occluding composition is disclosed in US 5,374,417 which discloses a potassium salt of a synthetic anionic polymer, such as a polycarboxylate.

WO 00/78270 describes an oral composition of arginine bicarbonate and calcium carbonate to promote the formation of dentinal tubule plugs aided by combining calcium and phosphate in the fluid.

There is a continuing need for desensitizing agents which can provide treatment and relief of hypersensitivity whilst maintaining a pleasing "mouth-feel" for the product in which they are formulated.

The present inventors have found that this problem may be solved by the use of certain particulate calcium carbonates of specific particle size and shape.

Summary of the Invention

The present invention provides a composition containing particulate calcium carbonate composed of primary particles which are scalenohedral and which have an average size of 2 microns or less; for the treatment of hypersensitivity arising in natural human teeth by application of the composition thereto.

The invention also provides a method of treating hypersensitivity arising in natural human teeth, the method comprising applying thereto an effective amount of a composition as described above.

The invention also provides the use of particulate calcium carbonate composed of primary particles which are scalenohedral and which have an average size of 2 microns or less; for the treatment of hypersensitivity arising in natural human teeth.

The scalenohedral calcium carbonate particles as defined above are effective as dentinal tubule occluding agents. Furthermore they can provide a smooth and pleasant mouth-feel when formulated into products such as dentifrices. Additionally, by reacting with phosphates (such as those naturally found in saliva), the scalenohedral calcium carbonate particles as defined above may assist with strengthening or remineralising enamel or dentine, and may help to form plugs in tubules which further reduce sensitivity.

Scalenohedral calcium carbonates have been proposed in the literature as a dentifrice component. However, there is no suggestion that scalenohedral calcium carbonates would be effective in the context now described, namely the treatment of hypersensitivity arising in the natural human tooth, and in particular the occlusion of dentinal tubules. ,

## DETAILED DESCRIPTION OF THE INVENTION

Particulate calcium carbonate

The composition for use in the invention comprises, *inter alia*, a particulate calcium carbonate composed of primary particles which have a specific shape and size, as defined above.

By "primary particles" is meant individual particles, defined as the smallest discrete particles that can be seen by electron microscopy analysis (such as, for example, individual crystals). For the purposes of the present invention, the primary particles of the particulate calcium carbonate are scalenohedral and have an average size of 2 microns or less.

The primary particles may associate under certain conditions to form larger secondary structures such as aggregates or agglomerates.

For the purposes of the present invention, a suitable source of particulate calcium carbonate as defined above includes crystalline calcium carbonates in which the individual crystals have a scalenohedral morphology and an average size of 2 microns or less.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline forms of calcium carbonate are available naturally, or may be synthetically produced in three particular crystalline morphologies, calcite, aragonite, and less commonly found, vaterite. The vaterite form of calcium carbonate is metastable and irreversibly transforms into calcite and aragonite. There are many different polymorphs (crystal habits) for each of these crystalline forms. The calcite crystalline morphology is the most commonly used crystal form of calcium carbonate. Over 300 crystalline forms of calcite have been reported in the literature.

References to a "scalenohedral crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are scalenohedral crystals. Scalenohedral crystals have a set of faces that are inclined to the vertical or "c"-axis direction, each of which are scalene triangles (i.e. triangles whose three sides are unequal in length). For the purposes of the present invention, preferred scalenohedral crystals have a trigonal scalenohedral shape, with twelve scalene triangle faces. Crystal shape may be determined by standard techniques known to those skilled in the art such as scanning electron microscopy (SEM). SEM is an imaging and analysis technique based on the detection of electrons and X-rays that are emitted from a material when irradiated by a scanning electron beam. Imaging allows the user to distinguish between primary particles and aggregates or agglomerates.

Usually, crystals grow in three directions, length, width and height. Some crystals however, have one or two preferred growth directions. For the purposes of the present invention, preferred scalenohedral calcium carbonate crystals are elongated with a ratio between the length and the width of the crystal (the socalled aspect ratio) of greater than 1:1, and typically from about 5:1 up to about 10:1 (length:width). The aspect ratio of a particle (e.g. a crystal) may typically be obtained from SEM photographs by averaging the ratio between the length and width of the particle, measured on at least 10 particles for 1 photograph.

For the purposes of the present invention, preferred scalenohedral calcium carbonate crystals have an average size which generally ranges from about 0.05 to about 1.5 microns, with a preferred size ranging from about 0.1 to about 1 micron, more preferably from about 0.15 to about 0.5 micron, most preferably from about 0.2 to 0.35 micron. A standard technique known to those skilled in the art for determining the average size of the individual crystals is air permeametry according to the Lea and Nurse method (standards NF X 11-601 and NF X 11 602). This analytical technique determines the average particle size by measuring the pressure drop across a packed powder bed using a water manometer. The pressure drop is a function of the permeability of the packed bed. This is related to the surface area of the particles which is then transformed to an average size based upon the assumption that the particles are spherical. Average particle size (Dp) is obtained from the massic area (SM) derived from the Lea and Nurse method by making the assumptions that all the particles are spherical, non-porous and of equal diameter, and by neglecting contact surfaces between the particles. The relationship between Dp and SM is the following:

$$Dp = 6/(\rho SM)$$

where p is the specific mass of the calcium carbonate. The average particle size (Dp) obtained in this way represents the average equivalent spherical diameter, or average ESD, where equivalent spherical diameter (ESD) is defined as the diameter of a notional sphere having the same volume as the particle.

For the purposes of the present invention, preferred scalenohedral calcium carbonate crystals may be further characterised as having a BET specific surface area higher than or equal to 0.1 $m^2$/g, preferably higher than or equal to 1 $m^2$/g, more preferably higher than or equal to 3 $m^2$/g and most preferably higher than or equal to 4 $m^2$/g. The calcium carbonate particles according to the invention generally have a BET specific surface area lower than or equal to 30 $m^2$/g, preferably lower than or equal to 25 $m^2$/g, more preferably lower than or equal to 20 $m^2$/g, and most preferably lower than or equal to 15 $m^2$/g. The BET specific surface area is usually measured according to the standard ISO 9277 which specifies the determination of the overall specific external and internal surface area of disperse or porous solids by measuring the amount of physically adsorbed gas according to the Brunauer, Emmett and Teller (BET) method.

As indicated above, primary particles (such as individual crystals) may associate under certain conditions to form larger secondary structures such as aggregates or agglomerates. Specifically, the scalenohedral calcium carbonate crystals described above may associate under certain conditions to form secondary structures. The size of these secondary structures is generally around Dp (as defined above) x 10, and may be determined by standard techniques known to those skilled in the art such as sedimentation. Particle size values obtained from sedimentation techniques are also normally expressed in terms of the equivalent spherical diameter (ESD), i.e. the diameter of a notional sphere having the same volume as the particle. The ESD value may be calculated based on the sedimentation rate of the particle in question as defined by Stokes' Law (Micromeritics SediGraph® 5100 Particle Size Analysis System Operator's Manual, V2.03, 1990). The sedimentation rate is measured using a finely collimated beam of low energy X-rays which pass through the sample cell to a detector. For a sample population of particles, the distribution of particle mass at various points in the cell affects the number of X-ray pulses reaching the detector. This X-ray pulse count is used to derive the particle size distribution expressed as the percent mass at given particle diameters. The median value of the ESD which can be derived from this distribution (i.e. the particular ESD value on the cumulative mass distribution curve at which 50 percent mass of the population has a higher ESD and 50 percent mass of the population has a lower ESD) is usually quoted as the average particle size of the sample population. Preferred scalenohedral calcium carbonate crystals for use in the invention may form secondary structures such as rosettes, clusters or "starburst" structures (i.e a multiplicity of primary particles emanating from a central core) with an average particle size (median ESD determined by sedimentation) ranging from about 2 to 3 microns.

Crystalline forms of calcium carbonate having scalenohedral crystal morphology (as described above) and suitable for use in the invention are available naturally, or may be produced by precipitation production technology. Typically, precipitated calcium carbonate is prepared by exposing calcium hydroxide slurry to a carbonation reaction. Control of the specific solution environment during the nucleation and growth of calcium carbonate governs the size and the shape of the crystals in the resulting precipitated calcium carbonate product.

For the purposes of the present invention, a particularly preferred class of particulate calcium carbonate includes crystalline calcium carbonates in which the individual crystals have a scalenohedral morphology and an average size of 2 microns or less, and also in which at least 50% by weight, preferably at least 80% by weight of the crystals are trigonal scalenohedral calcite crystals with an average size (Dp measured by air permeametry) ranging from about 0.2 to 0.35 micron and an aspect ratio ranging from about 5:1 up to about 10:1.

A commercially available source of particulate calcium carbonate suitable for use in the invention is SOCAL® S2E Precipitated Calcium Carbonate (PCC), ex Solvay S.A.

Mixtures of any of the above described materials may also be used.

In order to treat hypersensitivity arising in natural human teeth, the particulate calcium carbonate as defined above (hereinafter termed "scalenohedral calcium carbonate") will generally be incorporated into an oral care composition. Examples of such oral care compositions include dentifrice, mouthwash, tooth powder, chewing gum, lozenge or other oral care product form suitable for topical administration to teeth.

The relative amount of scalenohedral calcium carbonate to be incorporated into such a composition will depend to some extent on the type of composition used and its typical mode of use (for example typical product dosage and the amount of time it stays in contact with the affected site).

In dentifrices for use in the invention, for example, the amount of scalenohedral calcium carbonate may generally range from 1 to 50%, preferably from 10 to 40%, more preferably from 30 to 40%, by total weight scalenohedral calcium carbonate based on the total weight of the dentifrice.

A dentifrice represents a preferred type of oral care composition in the context of the present invention. The term "dentifrice" denotes a composition which is used to clean the surfaces of the oral cavity. The dentifrice is an oral composition that is not intentionally swallowed for purposes of systemic administration of therapeutic agents, but is retained in the oral cavity for a sufficient time to contact substantially all of the dental surfaces and/or mucosal tissues for purposes of oral activity. Preferably the dentifrice is suitable for application with a toothbrush and is rinsed off after use. Preferably the dentifrice is in the form of an extrudable semi-solid such as a cream, paste or gel (or mixture thereof).

A dentifrice for use in the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A dentifrice for use in the invention will also generally contain further ingredients to enhance performance and/or consumer acceptability.

For example, a dentifrice will generally contain a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectarites, colloidal magnesium aluminium silicates and mixtures thereof. However, an additional advantage of the scalenohedral calcium carbonate of the invention is that it may itself provide a thickening effect when incorporated into a dentifrice product form. This allows the level of binders or thickening agents (as defined above) to be reduced, for example to less than 5%, more preferably less than 1%, and most preferably less than 0.1% by weight based on the total weight of the dentifrice.

The dentifrice will also usually contain a surfactant in an amount of from 0.2 to 5% by weight based on the total weight of the dentifrice. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

An additional advantage of the scalenohedral calcium carbonate of the invention is that it may provide a smooth and pleasant mouth-feel when incorporated into a dentifrice product form, such as described above. This is particularly advantageous in the context of dental hypersensitivity treatment since it encourages regular and repeated product use by the consumer, which reinforces the anti-hypersensitivity benefit achieved.

A further advantage of the scalenohedral calcium carbonate of the invention is that it may play a dual role of desensitizer and gentle tooth whitener, when incorporated into a dentifrice product form, such as described above.

Accordingly, a preferred dentifrice for use in the invention demonstrates satisfactory levels of cleaning, yet is not unduly abrasive and damaging to the teeth, when measured for example as described below.

Such a dentifrice for use in the invention may comprise abrasive materials selected from abrasive silicas, calcium carbonates (which are different to the scalenohedral calcium carbonate described above), dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. However, it is preferred that the total amount of these abrasive materials is less than 5%, more preferably less than 1%, and most preferably less than 0.1 % by weight based on the total weight of the dentifrice.

The level of cleaning ability of a dentifrice for use in the invention may be measured by assessing its effect on accumulated pellicle, for example by using a method such as that described by Pickles et al.(International Dental Journal 55 (2005), pp. 197-202). This model uses enamel slabs cut from bovine central incisors, embedded in methacrylate resin. The enamel surfaces are smoothed by hand using an alumina paste on a glass block and lightly acid etched in order to facilitate stain accumulation and adherence. The enamel blocks are placed in an incubator set at a constant temperature of 50°C and slowly rotated to alternate between immersion in a staining broth (consisting of tea, coffee and mucin) and air drying. The broth is changed daily and after five days the slabs are removed and washed with distilled water to remove any loose debris. The stained slabs are then brushed in a mechanical brushing machine with distilled water to remove any loosely adhered stain. They are then dried and the L* values (L*stained) from the CIE L*a*b* colour system are measured with a chroma meter in L*a*b* mode. To assess cleaning performance, the stained specimens are then mounted in the mechanical brushing machine and the required load applied to each brush. The test composition is dispersed in an aqueous diluent to form a slurry (typically 38.5g test composition and 61.5g of distilled water) and the stained specimens brushed for a set number of brush strokes with 10ml of slurry. After brushing, the enamel slabs are rinsed with distilled water, dried and the L* values (L*brushed) are re-measured. In the final stage, all traces of stain are removed from the enamel slabs using flour of pumice, on a soft cloth using a grinder/polisher. The enamel slabs are then rinsed with distilled water, dried and the L* values (L*pumiced) are measured and recorded. The percentage of the stain removed by the test composition compared to full removal by pumice (hereinafter referred to as the pellicle cleaning ratio or PCR) may be calculated using the following equation:

$$PCR = [(L^*_{brushed})-(L^*_{stained})/ (L^*_{pumiced})-(L^*_{stained})] \times 100$$

A dentifrice for use in the invention preferably has a PCR value of at least 30%, more preferably at least 40%, most preferably at least 45%.

The level of abrasivity of a dentifrice for use in the invention may be measured by assessing its Radioactive Dentine Abrasion Test (RDA) value. A standard test procedure for measuring these values follows the method for assessment of dentifrice abrasivity recommended by the American Dental Association (Journal of Dental Research 55(4) 563, 1976). In this procedure, extracted human teeth are irradiated with a neutron flux and subjected to a standard brushing regime. The radioactive phosphorus 32 removed from the dentin in the roots is used as the index of the abrasion of the composition tested. A reference slurry containing 10 g of calcium pyrophosphate in 50 cm$^3$ of 0.5% aqueous solution of sodium carboxymethyl cellulose is also measured and the RDA of this mixture is arbitrarily taken as 100. In order to measure the RDA for the test composition, a slurry of 25g of the test composition in 40 cm$^3$ water is prepared and submitted to the same brushing regime.

A dentifrice for use in the invention preferably has a RDA value of no more than 100, more preferably no more than 80, most preferably no more than 60.

A dentifrice for use in the invention preferably has a PCR:RDA ratio of at least 0.5, more preferably at least 0.6, most preferably at least 0.8.

Compositions for use in the invention (such as in particular dentifrices) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

The invention is further illustrated with reference to the following, non-limiting Example.

### Claims

1. A composition containing particulate calcium carbonate composed of primary particles which are scalenohedral and which have an average size of 2 microns or less; for use in the treatment of hypersensitivity arising in natural human teeth.

2. A composition for use according to claim 1, in which the particulate calcium carbonate is selected from crystalline calcium carbonates in which the individual crystals are scalenohedral and have an average size of 2 microns or less.

3. A composition for use according to claim 2, in which at least 50% by weight, preferably at least 80% by weight of the crystals are trigonal scalenohedral calcite crystals with an average size ranging from about 0.2 to 0.35 micron and an aspect ratio ranging from about 5:1 up to about 10:1.

4. A composition for use according to any one of claims 1 to 3, which is in the form of a dentifrice and contains a liquid continuous phase in an amount of from 40 to 99% by weight based on the total weight of the dentifrice, the liquid continuous phase comprising a mixture of water and polyhydric alcohol.

5. A composition for use according to any one of claims 1 to 4, in which the level of the particulate calcium carbonate ranges from 30 to 40%, by total weight of the particulate calcium carbonate based on the total weight of the composition.

### Patentansprüche

1. Zusammensetzung, die teilchenförmiges Calciumcarbonat enthält, das aus primären Partikeln gebildet wird, die scalenoedrisch sind und die eine durchschnittliche Größe von 2 Mikrometern oder weniger aufweisen, zur Verwendung bei der Behandlung von Überempfindlichkeit, die in natürlichen menschlichen Zähnen auftritt.

2. Zusammensetzung zur Verwendung nach Anspruch 1, in der das teilchenförmige Calciumcarbonat aus kristallinen Calciumcarbonaten ausgewählt ist, in welchen die individuellen Kristalle scalenoedrisch sind und eine durchschnittliche Größe von 2 Mikrometern oder weniger aufweisen.

3. Zusammensetzung zur Verwendung nach Anspruch 2, in welcher mindestens 50 Gewichts-%, vorzugsweise mindestens 80 Gewichts-% der Kristalle trigonale scalenoedrische Calcitkristalle mit einer durchschnittlichen Größe von etwa 0,2 bis 0,35 Mikrometern und mit einem Seitenverhältnis von etwa 5:1 bis zu etwa 10:1 sind.

4. Zusammensetzung zur Verwendung nach irgendeinem der Ansprüche 1 bis 3, die in Form eines Zahnputzmittels vorliegt und eine flüssige kontinuierliche Phase in einer Menge von 40 bis 99 Gewichts-%, bezogen auf das Gesamtgewicht des Zahnputzmittels enthält, wobei die flüssige kontinuierliche Phase eine Mischung von Wasser und mehrwertigem Alkohol umfasst.

5. Zusammensetzung zur Verwendung nach irgendeinem der Ansprüche 1 bis 4, in welcher sich der Anteil des teilchenförmigen Calciumcarbonats von 30 bis 40% des Gesamtgewichts des teilchenförmigen Calciumcarbonats, bezogen auf das Gesamtgewicht der Zusammensetzung, erstreckt.


**Revendications**

1. Composition contenant du carbonate de calcium particulaire constituée de particules primaires qui sont scalénoèdriques et qui présentent une taille moyenne de 2 microns ou inférieure ; pour une utilisation dans le traitement de l'hypersensibilité provenant de dents naturelles de l'être humain.

2. Composition pour une utilisation selon la revendication 1, dans laquelle le carbonate de calcium particulaire est choisi parmi des carbonates de calcium cristallins dans lesquels les cristaux individuels sont scalénoèdriques et présentent une taille moyenne de 2 microns ou inférieure.

3. Composition pour une utilisation selon la revendication 2, dans laquelle au moins 50 % en masse, de préférence au moins 80 % en masse des cristaux sont des cristaux de calcite scalénoèdriques trigonaux avec une taille moyenne d'environ 0,2 à 0,35 micron et un rapport d'allongement d'environ 5:1 jusqu'à environ 10:1.

4. Composition pour une utilisation selon l'une quelconque des revendications 1 à 3, laquelle est dans la forme d'un dentifrice et contient une phase continue liquide dans une quantité de 40 à 99 % en masse rapportée à la masse totale du dentifrice, la phase continue liquide comprenant un mélange d'eau et d'alcool polyvalent.

5. Composition pour une utilisation selon l'une quelconque des revendications 1 à 4, dans laquelle la teneur du carbonate de calcium particulaire est de 30 à 40 %, en masse totale du carbonate de calcium particulaire rapportée à la masse totale de la composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5270031 A
- US 5374417 A
- WO 0078270 A

### Non-patent literature cited in the description

- **PICKLES et al.** *International Dental Journal,* 2005, vol. 55, 197-202
- *Journal of Dental Research,* 1976, vol. 55 (4), 563